# EUROPEAN PATENT SPECIFICATION

(11) **EP 1 709 987 B1**
(45) Date of publication and mention of the grant of the patent: **23.12.2009**
(21) Application number: 05075800.2
(22) Date of filing: 07.04.2005
(51) Int. Cl.: A61M 25/01

(54) **Steerable catheter assembly**
Lenkbare Katheteranordnung
Ensemble de cathéter dirigeable

(43) Date of publication of application: 11.10.2006
(73) Proprietor: Creganna Technologies Limited, Ballybrit, Galway (IE)
(72) Inventor: Corcoran, Neil, County Cavan (IE); O'Reilly, Dermot, Killarney, County Kerry (IE)
(74) Representative: Shortt, Peter Bernard

(56) References cited:
- WO-A-2004/009150
- US-A1- 2003 120 259

## Description

### Field of the Invention

The invention relates to steerable catheters for use in minimally invasive surgical procedures.

### Background to the Invention

Catheters for insertion into bodily lumens, e.g. intravascular catheters and the like are well known in the art. Catheters typically employ elongate flexible tubes made from a synthetic plastics material or from stainless steel. Desirable characteristics of catheter tubing include "pushability", that is the ability to transfer forces from the proximal to the distal end of the catheter. It is also an advantage for the catheter to have good "trackability", i.e. to be sufficiently flexible as to be capable of navigating tortuous paths within a body lumen without kinking. It is also desirable for the catheter to have good "torqueability", as manipulation of a device within a body lumen often requires small precise amounts of torque to be applied.

Non-steerable delivery devices such as hypotubes are widely used for delivery of devices in a variety of minimally invasive surgical procedures. Some examples of typical devices that may be delivered to a pre-determined area are stents and balloons. Stents may be used to hold an artery wall open where a constriction or stenosis has occurred. Repeated inflation and deflation of balloons may be used to detach plaque from artery walls. The term hypotube refers generally to a metallic tube or shaft (although plastics may also be used), preferably of stainless steel, having a lumen extending therethrough. The tube or shaft is preferably of thin-walled construction.

Metals are generally used as they provide several advantages to the surgeon when he or she delivers a device or treatment percutaneously, to locations within any lumen of the human body, including the vasculature, the bilary system, oesophagus, and gastrointestinal tract. A first advantage is the degree of pushability of the hypotube through the vasculature of the human body. Pushability may be defined as the ability to transfer energy from one end of the hypotube shaft to the other. Pushability is therefore a measure of the transfer of the force from the proximal end to the distal end of the hypotube, that is, the ratio of force applied at the proximal end by the physician as compared to the force measured at the distal end. Good pushability means that force is efficiently and effectively transferred through the hypotube.

Another advantage is the torque or torqueability provided by the hypotube. Torqueability may be defined as the ability to transmit a rotational displacement along the length of the hypotube shaft. Perfect torqueability means that one turn at the proximal end of the hypotube would result in one turn at the distal end - this is often represented as 1:1 torque.

A further advantage is the kink resistance provided by metal hypotubes. Kink resistance may be defined as the shaft's ability to maintain its cross sectional profile during deformation.

Hypotubes also provide a usable inner lumen, which may be used for delivery of inflation fluids to catheter balloons, delivery of contrast media to a site within the human body, delivery of drugs for localised treatment and a range of other applications. Furthermore, hypotubes are capable of producing exceptionally low profiles to tight tolerances. This ensures that the procedure is a comfortable experience for the patient. Hypotubes are also relatively low in cost.

Steerable mechanisms are also widely available for catheter delivery and navigation in a variety of minimally invasive surgical procedures. They may be used to deliver or push a catheter or other device, such as a pacemaker lead, through tortuous anatomy or to locate the entrance to an artery or vein.

Prior art steerable devices have often sacrificed many of the advantages of non-steerable delivery devices such as hypotubes. Typical steering mechanisms involve the use of a pull wire, which is connected to the distal end of the catheter tip. When a tensile force is applied to the pull wire, the tip of the catheter will bend proportionally in response to the force applied. Since the wire must be contained and controlled in some way, these steerable devices usually include a dedicated lumen to enclose the pull wire. This has the disadvantage of increasing manufacturing costs of the steerable device. It also has the disadvantage of adding weight to the product and increasing the overall profile of the device, thus making the procedure more uncomfortable for the patient. In addition, relatively complex handles are required in order to control tension in the pull wire. These are often bulky, with poor ergonomics and can be costly to manufacture. Furthermore, the pull wires are used under tension, and there is thus a possibility of breakage of the pull wires under tension during use.

US Patent No. 6,783,510 relates to a steerable catheter including a single pull wire arranged to allow the catheter to achieve various complex curvatures. The pull wire extends through two different offset lumens and attaches to the distal end of the catheter at an off-axis location. By tensioning the pull wire, the catheter can assume various complex curves, depending on the respective lumen through which the pull wire passes. A further disadvantage with this type of catheter is that the tip of the catheter may only be deflected in a single plane. This restriction limits the effectiveness of this type of catheter in reaching many of the desired treatment sites.

Steerable catheter devices often involve use of polymer extrusions. As described above, steerable systems that include pull wires require a separate lumen for each pull wire. Polymer extrusions are often used to create tubes with lumens as they may be mass-produced more cheaply than metallic tubes with lumens. Due to the fact that the material yield strength, and thus the column strength of a polymer tube is significantly lower than that of a steel tube, use of such extrusions is likely to result in a delivery device with lower pushability and torqueability than hypotubes. In an attempt to address this shortcoming, some manufacturers have used a braided polymer. A braided polymer comprises a metal braid embedded in a polymer jacket. However, the resulting device still does not perform as well as a metal hypotube. This type of arrangement may also lead to 'springback' in the device. Springback occurs when the inherent elasticity and memory of the polymer materials results in the polymer tube creeping away from a desired shaped or bent position towards its original straight position. This can make small, accurate movements difficult to achieve.

If a device were to provide perfect torque (or 1:1 torque), steerability would only be required in a single plane, as all other planes could be accessed by rotational displacement of the device. Due to decreased levels of torque provided by prior art steerable devices, some manufacturers have attempted to increase the steerability to compensate for lack of torqueability.

This may be done by introducing more pull-wires to steer in multiple planes, as described in US Patent No. 5,383,852. This relates to a steerable catheter with independent proximal and distal control. The catheter has an elongate flexible shaft and a flexible tip assembly connected to the distal end of the shaft. The tip assembly comprises a distal section and a proximal section coaxial with the shaft. The stiffness and length of the distal and proximal sections are selected to provide a predetermined curve configuration of the tip assembly when the distal section or proximal section is bent. The catheter includes two pairs of pull cables, which extend through the catheter. The first pair of cables is anchored at a first point in the tip assembly for bending the proximal section in a predetermined plane. The second pair of cables is anchored at a second point in the tip assembly for bending the distal section in a predetermined plane. This allows any desired orientation of the bending planes to be achieved. A handle or actuator is used to apply tension to the pull cables in order to control the deflection of the tip assembly. However, including additional pull wires in the assembly has the effect of compounding the original problems of large profile devices, high manufacturing costs and more complex handles.

Many of these devices sacrifice the working inner lumen in order to keep the diameter low. Since the capability to deliver a device or a treatment through the lumen is now lost, a second device may be necessary to carry out this function. This results in longer surgical procedures, increased material cost per procedure and increased risk to the patient. Another disadvantage of the loss of the working lumen is that the surgeon can no longer track the catheter over a guidewire, which is a widespread practice in percutaneous, minimally invasive procedures.

US Patent No. 6,802,835 relates to a steerable catheter device comprising an outer sheath, which engages a handle, and a flexible catheter with a shape memory tip which also engages the handle and which extends through the sheath. In a fully extended position, the memory tip extends at substantially 90 degrees to the longitudinal axis of the device. As the tip is withdrawn into the sheath from the fully extended position, the angle of the tip relative to the longitudinal axis can be varied infinitely between about 90 degrees and 0 degrees. When the tip is withdrawn so as to be entirely contained within the sheath, the tip is substantially aligned with the longitudinal axis of the catheter. One disadvantage of this steerable catheter is that longitudinal movement of one shaft relative to the other is required. This means that a device, such as a stent or balloon, loaded at the distal end of the catheter would prevent the necessary movement of the memory tip. For this reason, the catheter device may not be suitable for placing a stent or balloon within a body vessel of a patient. Furthermore, as the tip of the device is flexible, pushing a rigid device such as an ablation catheter through the inner member would be likely to force the flexible tip to straighten.

International Patent Publication No. WO2004/009150 describes the closest prior art and relates to a telescopic introducer with a compound curvature for inducing alignment. The introducer comprises a flexible elongate outer sheath, and a flexible elongate telescopic inner sheath or core, telescopically disposed in the outer sheath. The outer and inner sheaths are rotatable relative to one another. A portion of the outer element has a first shape and a more proximal portion of the inner element also has the first shape. The inner element has a second shape on its distal portion. When the portions of the inner and outer elements having matching first shapes are aligned, the distal portion of the inner element will be extending in a predetermined selected orientation as intended for access to a desired portion of the coronary sinus branch venous system.

It is therefore desirable to provide a steerable catheter device, which includes the desirable properties of a hypotube delivery device, such as pushability, torqueability and kink resistance. It is also desirable to provide a steerable device which may be controlled more simply than prior art devices. It is also desirable to provide a steerable catheter device with a working inner lumen.

### Object of the Invention

An object of the invention is to provide a steerable catheter, which includes the desirable properties of a hypotube delivery device such as pushability, trackability, kink resistance and torqueability. It is also an object to provide a steerable catheter with a working lumen. Another object is to provide a single use steerable catheter which is lightweight and inexpensive to manufacture.

### Summary of the Invention

The present invention provides a steerable catheter assembly, comprising an elongate outer shaft having a lumen therethrough and having a proximal end and a distal end; an elongate inner shaft coaxially disposed within the lumen of the outer shaft and having a proximal end and a distal end; and characterised in that at least one of the inner shaft and the outer shaft is formed with a curvature at a distal portion, such that in an aligned configuration, a distal end of the catheter assembly is disposed at a maximum deflection angle α to a longitudinal axis of the catheter assembly; and the inner shaft and the outer shaft are rotatable relative to one another out of the aligned configuration such that one shaft exerts a deflection force on the other shaft to deflect the distal end of the catheter assembly between α and 0 degrees to the longitudinal axis of the catheter assembly.
In one embodiment of the invention, the inner shaft includes an inner lumen extending therethrough. In other embodiments, the inner shaft may comprise a solid bar or mandrel, which may be made from a flexible metal, shape memory metal or plastics material.

Preferably, the outer shaft containing the lumen has a high column strength. The outer shaft is typically metallic. Preferably, the outer shaft is formed from stainless steel. Alternatively, the outer shaft may be formed from nitinol, a cobalt based alloy, or a suitable polymer. Preferably, the inner shaft has a high column strength. The inner shaft is typically metallic. Preferably, the inner shaft is formed from stainless steel. Alternatively, the inner shaft may be formed from nitinol, a cobalt based alloy, or a suitable polymer.An advantage of the catheter assembly of the present invention is that it provides the desirable properties of a hypotube, such as pushability, trackability, torqueability and kink resistance, while also providing the steerability normally associated with more complex pull wire systems. Another advantage of the catheter assembly of the present invention is that it a working inner lumen may be provided. According to an embodiment of the invention, the inner shaft is formed with a curvature at a distal portion, and the outer shaft is formed with a curvature at a distal portion, and in the aligned configuration, the distal portion of the inner shaft is substantially aligned with the distal portion of the outer shaft, such that the distal end of the catheter assembly is disposed at a maximum deflection angle α to a longitudinal axis of the catheter assembly; and the inner shaft and the outer shaft are rotatable through an angle of rotation which may be in the range of 0° to about 180° relative to one another so that the distal portion of the inner shaft is moved out of alignment with the distal portion of the outer shaft, such that each shaft exerts a deflection force on the other shaft to deflect the distal end of the catheter assembly between α and 0 degrees to the longitudinal axis of the catheter assembly. In one embodiment the angle of relative rotation of the shafts is between 0° and about 45°, but more typically is between 0° and about 90°. Preferably the path of relative rotation lies in a single plane.

According to another embodiment of the present invention, the inner shaft is formed with a curvature at a distal portion, and the distal portion of the outer shaft is capable of bending in a single pre-determined bend plane only, and in the aligned configuration, the curvature of the distal portion of the inner shaft is substantially aligned with the bend plane of the distal portion of the outer shaft, such that the distal end of the catheter assembly is disposed at a maximum deflection angle α to a longitudinal axis of the catheter assembly; and the inner shaft and the outer shaft are rotatable relative to one another so that the distal portion of the inner shaft is moved out of alignment with the bend plane of the distal portion of the outer shaft to deflect the distal end of the catheter assembly between α and 0 degrees to the longitudinal axis of the catheter assembly.
The angle of rotation may be in the ranges stated above. Ideally, the outer shaft comprises a relatively rigid material, and a plurality of slots are formed in a portion of the wall of the outer shaft in a substantially spiral or circumferential path to allow a distal portion of the outer shaft to bend in a single pre-determined bend plane only.

According to a further embodiment of the present invention, the outer shaft is formed with a curvature at a distal portion, and the distal portion of the inner shaft is capable of bending in a single pre-determined bend plane only, and in the aligned configuration, the curvature of the distal portion of the outer shaft is substantially aligned with the bend plane of the distal portion of the inner shaft, such that the distal end of the catheter assembly is disposed at a maximum deflection angle α to a longitudinal axis of the catheter assembly; and the inner shaft and the outer shaft are rotatable relative to one another so that the distal portion of the outer shaft is moved out of alignment with the bend plane of the distal portion of the inner shaft to deflect the distal end of the catheter assembly between α and 0 degrees to the longitudinal axis of the catheter assembly. The angle of rotation may be in the ranges stated above. Ideally, the inner shaft comprises a relatively rigid material, and a plurality of slots are formed in a portion of the wall of the inner shaft in a substantially spiral or circumferential path to allow a distal portion of the inner shaft to bend in a single pre-determined bend plane only.

According to yet another embodiment of the present invention, the outer shaft and the inner shaft are displaceable longitudinally relative to one another out of the aligned configuration, such that each shaft exerts a deflection force on the other shaft to deflect the distal end of the catheter assembly between α and 0 degrees to the longitudinal axis of the catheter assembly.

In an embodiment of the invention, the distal portion of the inner shaft, or the distal portion of the outer shaft, or the distal portions of both shafts may comprise a shape memory material.

In another embodiment of the invention, the distal portion of the inner shaft, or the distal portion of the outer shaft or the distal portion of both shafts may comprise a shaped metal coil or spring.

According to yet another embodiment of the present invention, at least one spacer is disposed in the lumen of the outer shaft between the outer shaft and the inner shaft, such that an intermediate working lumen is established therebetween. An advantage of this arrangement is that a second working lumen is thus available for use during the surgical procedure. The second working lumen may be used for delivery of instruments to a site within the body, delivery of contrast media or for inflation of a balloon catheter. An additional advantage is that concentricity between the inner and outer shafts is maintained.

According to another aspect of the invention there is provided a steerable catheter assembly, comprising an elongate outer shaft having a lumen therethrough and having a proximal end and a distal end; an elongate inner shaft coaxially disposed within the lumen of the outer shaft and having a proximal end and a distal end; and characterised in that the distal portion of the inner shaft is capable of bending in a pre-determined bend plane only, and the distal portion of the outer shaft is capable of bending in a pre-determined bend plane only, and in an aligned configuration, the bend plane of the distal portion of the inner shaft is substantially aligned with the bend plane of the distal portion of the outer shaft, such that a distal end of the catheter assembly is capable of bending in the pre-determined bend plane with a maximum flexibility; and the inner shaft and the outer shaft are rotatable relative to one another out of the aligned configuration so that the bend plane of the distal portion of the inner shaft is moved out of alignment with the bend plane of the distal portion of the outer shaft to vary the flexibility of the distal end of the catheter assembly between the maximum flexibility and a minimum flexibility.

In one embodiment, a plurality of slots are formed in a portion of the wall of the outer shaft in a substantially spiral or circumferential path to allow a distal portion of the outer shaft to bend in a pre-determined bend plane only. A plurality of slots may be formed in a portion of the wall of the inner shaft in a substantially spiral or circumferential path to allow a distal portion of the inner shaft to bend in a pre-determined bend plane only.

An advantage of this arrangement is that when inserting a device in a body lumen of a patient, such as for example, a pacemaker lead, is that the catheter assembly may be stiffened when it is required to push the device through a blockage, and may be made more flexible when it is required to navigate through the tortuous pathways of the vasculature. Prior art systems require separate stiff and flexible styluses to be used, wherein each stylus is alternately inserted and withdrawn from the body as required.

Several embodiments of the steerable catheter assembly of the prerent invention will now be described with reference to and/or as illustrated in the accompanying drawings.

### Brief Description of the Drawings

Figure 1 is a side elevation of a first embodiment of the steerable catheter of the present invention;
Figure 2 is a side elevation of the steerable catheter assembly of Figure 1, in which the inner shaft has been rotated relative to the outer shaft;
Figure 3 is a perspective view of one embodiment of an inner shaf: of the steerable catheter assembly of the present invention;
Figure 4 is a longitudinal cross section of the steerable catheter assembly of Figure 1;
Figure 5 is a longitudinal cross section of a further embodiment of the steerable catheter assembly of the present invention;
Figure 6A is a side elevation of an example of a steerable catheter, which is not within the scope of the claims,
Figure 6B is a perspective view of the steerable catheter of Figure 6A;
Figure 7A is a plan view of a portion of a shaft according to one embodiment of the present invention, in a laid flat state to facilitate understanding of the configuration of the slots;
Figure 7B is a perspective view of the portion of Figure 7A;
Figure 8A is a side elevation of a one embodiment of the steerable catheter assembly of the present invention (in the straightened position);
Figure 8B is a perspective view of the steerable catheter assembly of Figure 8A;
Figure 9 is a side elevation of a second embodiment of the steerable catheter assembly according to the present invention;
Figure 10 is a side elevation of a third embodiment of the steerable catheter assembly of the present invention;
Figures 12A and 12B are side elevations of a further example of a steerable catheter assembly;
Figures 13 and 13A arc side elevations of a further example of a s steerable catheter assembly, which is not within the scope of the claims;
Figure 14 is a side elevation of a further embodiment of the steerable catheter assembly of the present invention;
Figure 15A is a perspective view of a steerable catheter assembly according to a second aspect of the invention, in an aligned position, which is not within the scope of the claims;
Figure 15B is a perspective view of the steerable catheter assembly of Figure 15A, moved out of the aligned position; and
Figure 16 is a side elevation of a shaft for use in an embodiment of the steerable catheter assembly according to the present invention.

### Detailed Description of the Drawings

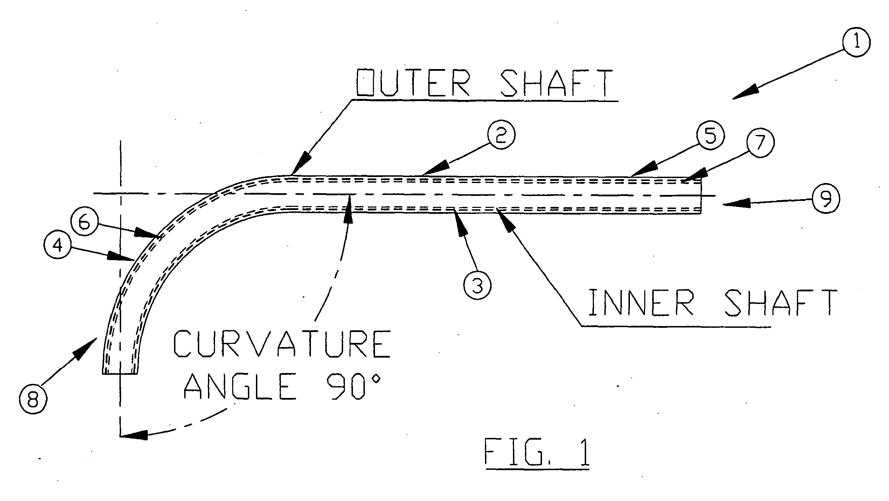

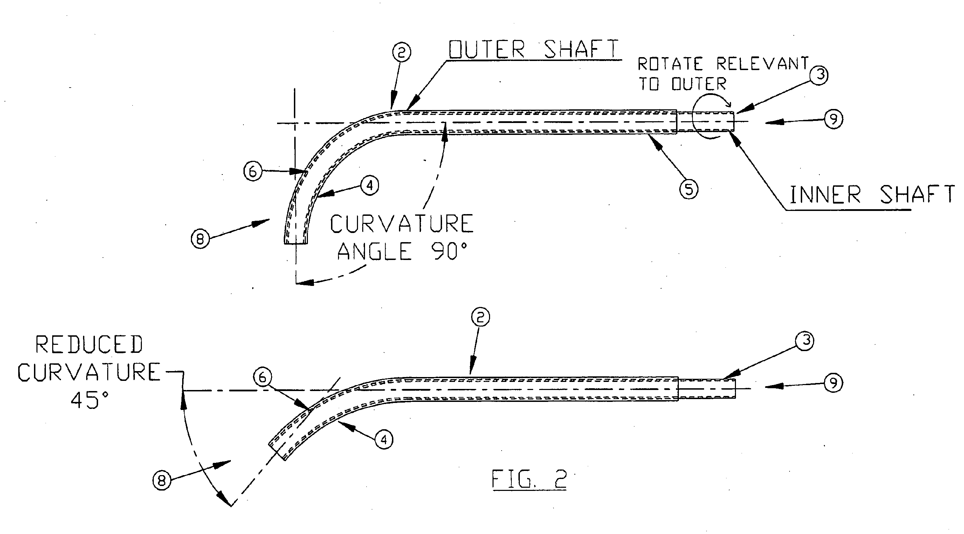

A first embodiment of the steerable catheter assembly of the present invention is shown in Figures 1 and 2. The catheter assembly 1 comprises a stainless steel outer shaft 2, having a lumen extending therethrough and a stainless steel inner shaft 3, having a lumen extending therethrough. Each of the shafts is of thin-walled construction. In alternative embodiments, the shafts may be formed from other metals, or from suitable plastics. The inner shaft is coaxially disposed within the outer shaft.

The outer shaft 2 is formed with a bend (or curvature) of approximately 90 degrees at a distal portion 4. The bend or curvature may be formed, for example, by heat treatment of the shaft. In an 'at rest' or unstressed position, the outer shaft distal end 4 will be disposed at an angle of approximately 90 degrees to the proximal end 5 of the shaft.
The bend or curvature is such that if the outer shaft is manually straightened, the shaft will automatically return to its unstressed position on release. The amount of curvature in the distal end 4 of the outer shaft 2 in the unstressed position (90 degrees in the present embodiment) represents the maximum steering angle of the catheter assembly 1.

Similarly, the inner shaft 3 is formed with a bend (or curvature) of approximately 90 degrees at a distal portion 6. In an "at rest" or unstressed position, the inner shaft distal end 6 will be disposed at an angle of approximately 90 degrees to the proximal end 7 of the shaft. The bend or curvature is such that if the inner shaft 3 is manually straightened, the shaft will automatically return to its unstressed position on release.

In a first configuration shown in Figure 1, the distal portion 4 of the outer shaft 2 is substantially aligned with the distal portion 6 of the inner shaft 3, such that the catheter assembly 1 has a curvature of approximately 90 degrees at its distal end 8. In the present embodiment, the catheter assembly may be adjusted between angles of 0 degrees and 90 degrees. In alternative embodiments, other angles of curvature may be used.

As shown in Figure 2, the steering mechanism works by effecting relative rotational displacement between the inner and outer shafts. In the at rest position shown in Figure 1, before any rotation takes place the inner shaft 3 and outer shaft 2 are aligned and the catheter assembly distal end 8 is disposed at an angle to the assembly proximal end 9. This angle is the 'at rest' angle formed by the shaft distal ends with the shaft proximal ends. In the embodiment shown in Figures 1 and 2, the outer shaft 2 is clamped at its proximal end 5 so that it cannot rotate. The inner shaft 3 is freely rotatable within the outer shaft. If the inner shaft is rotated, the bend at the inner shaft distal portion 6 is no longer aligned with the bend at the outer shaft distal portion 4. This results in a force being exerted on the outer shaft distal end 4 by the inner shaft distal end 6, which causes the outer shaft distal end 4 (and thus the catheter assembly distal end 8) to begin to straighten. If the inner shaft 3 is rotated, for example, through about 180°, the inner shaft distal portion 6 will be fully misaligned with the outer shaft distal portion 4, such that the curvature in the inner shaft distal end 6 is disposed in an opposite direction to that in the outer shaft distal end 4. The forces exerted by each shaft on the other are equal and opposite and the catheter assembly is caused to straighten. By rotating the inner shaft relative to the outer different angles of curvature (between 90 and 0 degrees) of the catheter assembly distal end 8 may be achieved. In alternative embodiments, the outer shaft is rotated while the inner shaft is held in place, or each shaft may be rotated by up to about 90 degrees in opposite directions.

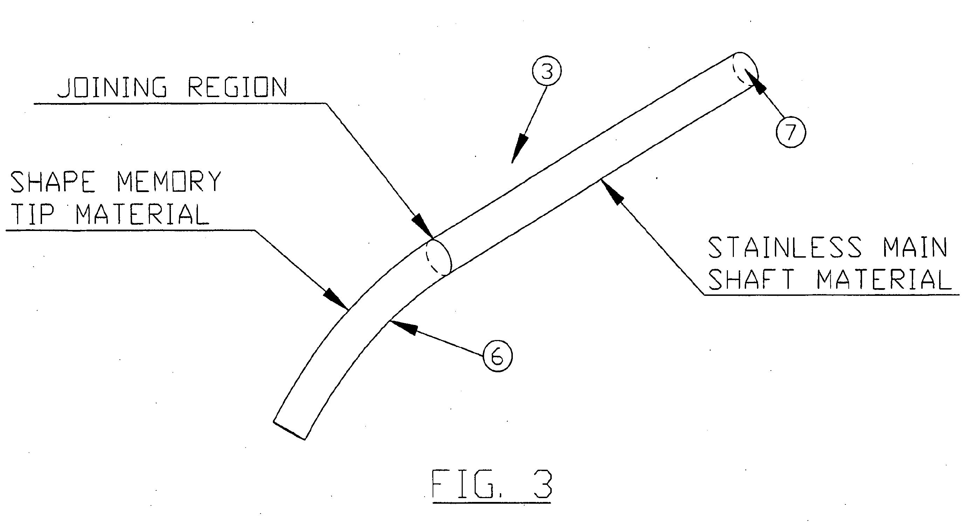

In the embodiment shown in Figure 3, the distal portion 6 of the inner shaft 3 comprises a shape memory material (such as nitinol). Nickle-based, copper-based, iron-based, platinum-based or polymer shape memory materials may also be used. Shape memory materials offer excellent elasticity, but may be relatively expensive. For this reason, the shape memory material is used only for the distal portion 6 of the inner shaft 3. The shape memory section is attached to the proximal shaft using a mechanical fit, a weld or other known joining method. The catheter assembly operates as described above. In alternative embodiments, the distal portion of the outer shaft is formed from a shape memory material, or the distal portions of both the inner and outer shafts are formed from a shape memory material.

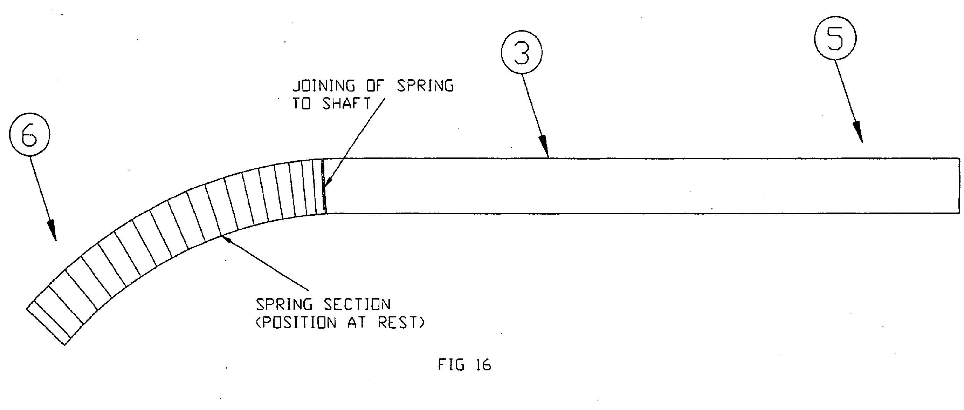

An alternative shaft for use in an embodiment of the present invention is shown in Figure 16. In this embodiment, the distal portion 6 of the inner shaft 3 (or of the outer shaft 2 or of both the inner and outer shafts) comprises a metal coil. This provides a cheaper alternative to the shape memory material. The metal coil is similar to a spring and may be formed with a curvature as previously described. The metal coil is attached to the proximal shaft by a mechanical fit, a weld or other known joining method.

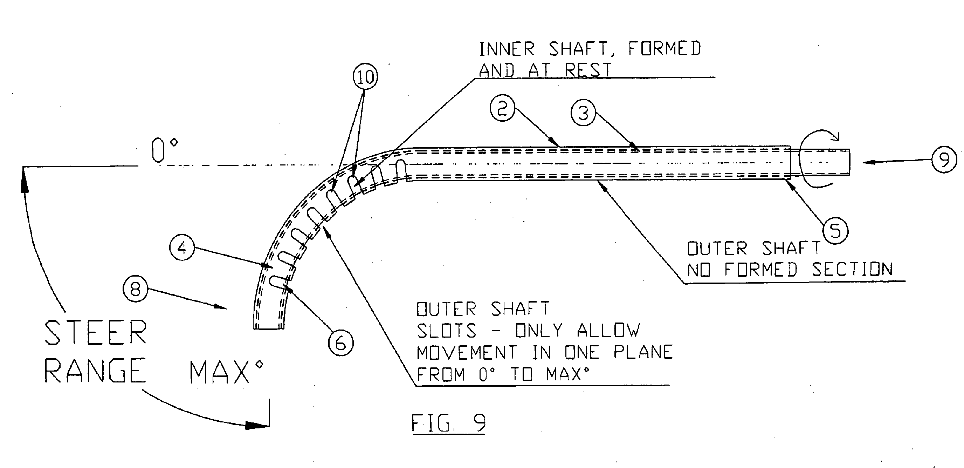

A second embodiment of the steerable catheter assembly of the present invention is shown in Figure 9. The inner shaft 3 is formed with a curvature of approximately 90 degrees at a distal end 6. This curvature may be formed, for example, by heat treatment of the shaft 3 or by use of shape memory material as described above with reference to Figure 3. The outer shaft 2 is not pre-formed with a bend or curvature but has had material removed at a distal portion 4 to form slots 10 on one side of the shaft 2. The outer shaft is formed from a material such as stainless steel and the slots 10 allow the outer shaft 2 to bend in one direction only. The slots render the shaft 2 sufficiently flexible to allow it to adopt the same shape as the inner shaft 3. In the 'at rest' position, the catheter assembly distal end 8 is therefore disposed at an angle of approximately 90 degrees to the catheter assembly proximal end 9. In this embodiment, the outer shaft 2 is clamped at its proximal end 5 so that it cannot rotate, whereas the inner shaft 3 is freely rotatable. When the inner shaft is rotated relative to the outer shaft, the outer shaft cannot deflect in any plane other than the single direction permitted by the slots, and the outer shaft thus begins to straighten. If the inner shaft is rotated through about 90 degrees, it exerts a deflection force on the outer shaft in the direction of curvature of the inner shaft. However, the rigidity of the outer shaft ensures that the outer shaft cannot be deflected (in any plane other than that allowed by the slots) beyond a straight position.

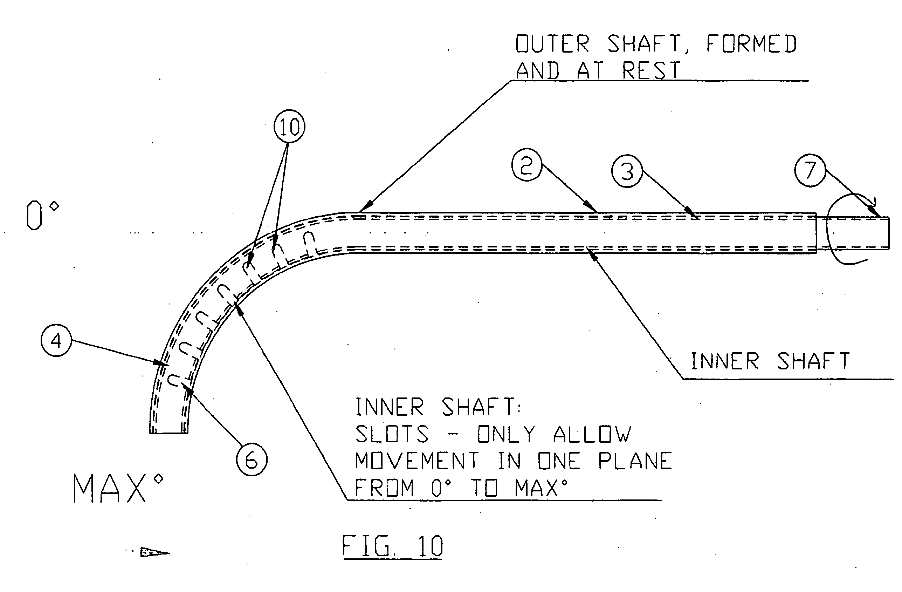

A third embodiment is shown in Figure 10. In this embodiment, the outer shaft 2 is formed with a curvature of approximately 90 degrees at its distal end 4 as described above with reference to Figures 1 to 3. The inner shaft 3 is not formed with a bend or curvature but has had material removed at a distal portion 4 to form slots 10 on one side of the shaft 2. The inner shaft 3 is formed from a material such as stainless steel and the slots 10 allow the inner shaft 3 to bend in one direction only. The slots render the shaft 3 sufficiently flexible to allow it to adopt the same shape as the outer shaft 2. In the at rest' position, the catheter assembly distal end 8 is therefore disposed at an angle of approximately 90 degrees to the catheter assembly proximal end 9. In this embodiment, the inner shaft 3 is clamped at its proximal end 7 so that it cannot rotate, whereas the outer shaft 2 is freely rotatable. When the outer shaft is rotated relative to the inner shaft, the inner shaft cannot deflect in any plane other than the single direction permitted by the slots, and the inner shaft thus begins to straighten. If the outer shaft is rotated through about 90° degrees, it exerts a deflection force on the inner shaft in the direction of curvature of the outer shaft. However, the rigidity of the inner shaft ensures that the inner shaft cannot be deflected (in any plane other than that allowed by the slots) beyond a straight position.

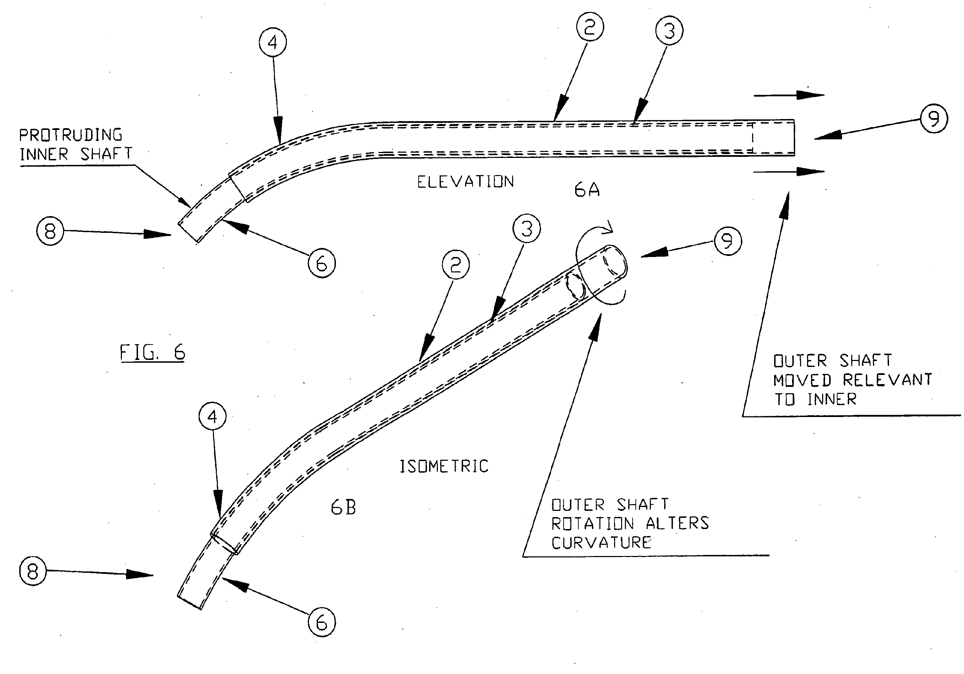

An example of a steerable catheter assembly 1 is shown in Figures. 6A and 6B. In this example, both the inner and outer shafts 3, 2 are formed with a bend or curvature of approximately 90 degrees at their distal ends 6, 4 as previously de scribed with reference to Figures 1 to 3. In the 'at rest' position, the distal end 8 of the catheter assembly 1 is disposed at approximately 90 degrees to the proximal end 9 of the assembly. The shape of the curvature of the assembly may be varied by effecting relative longitudinal movement between the inner and outer shafts as shown in Figure 6A. Longitudinal displacement may be combined with relative rotational displacement as described above.

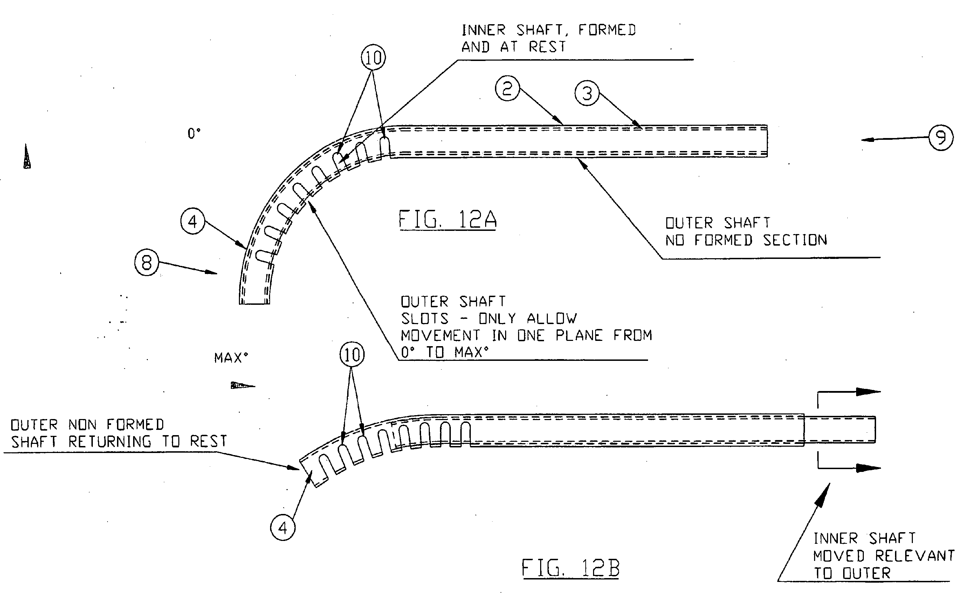

Another example the steerable catheter assembly 1 is shown in Figure 12. In this example, the inner shaft 3 is formed with a curvature of approximately 90 degrees at a distal end 6 as described above with reference to Figures 1 to 3. The outer shaft 2 is not pre-formed with a bend or curvature but has had material removed at a distal portion 4 to form slots 10 on one side of the shaft 2, as described above with reference to Figure 9. In the 'at rest' position, the catheter assembly distal end 8 is therefore disposed at an angle of approximately 90 degrees to the catheter assembly proximal end 9. If the inner shaft is moved longitudinally relative to the outer shaft, in a proximal direction, the distal end 4 of the outer shaft 2 will begin to straighten as the deflection force exerted on it by the inner shaft is removed.

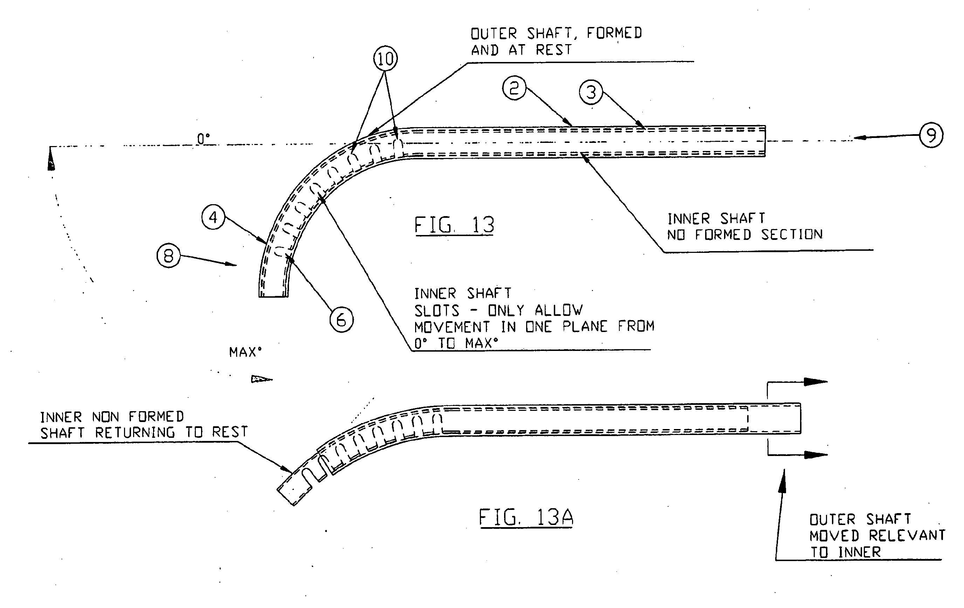

A further example is shown in Figures 13 and 13A, in which the outer shaft 2 is formed with a curvature of approximately 90 degrees at its distal end 4 as described above with reference to Figures 1 to 3. The inner shaft 3 is not formed with a bend or curvature but has had material removed at a distal portion 4 to form slots 10 on one side of the shaft 2 as described above with reference to Figure 10. In the 'at rest' position, the catheter assembly distal end 8 is therefore disposed at an angle of approximately 90 degrees to the catheter assembly proximal end 9. If the outer shaft is moved longitudinally relative to the inner shaft, in a proximal direction, the distal end 6 of the inner shaft 3 will begin to straighten as the deflection force exerted on it by the outer shaft is removed.

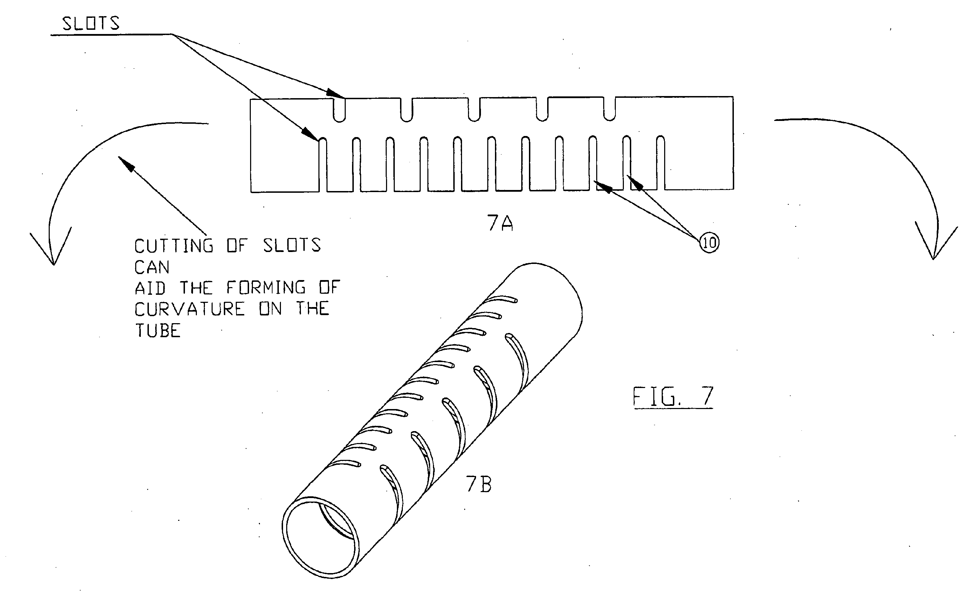

In a further embodiment of the present invention, as shown in Figure 7A and 7B, slots may be cut into the distal end of the inner shaft, the outer shaft or both shafts, in order to allow the shaft to bend or curve in a pre-determined direction. As described above with reference to Figures 9 and 10, slotting may be used to ensure that the shaft is only capable of bending in a single plane or direction. As shown in Figure 7, slots may be cut into both sides of the shaft in order to ensure that the shaft bends in a pre-determined bend plane.

Slots may also be used to vary the stiffness of the shafts. For example, a spiral (or helical) slot may be cut into the wall of the shaft as shown in Figues 8A and 8B. The pitch of the spiral determines the degree of flexibility imparted to the shaft.

In general, as indicated above, the relative rotation of the shafts can be through an angle of rotation in the range 0° and about 180°, but typically through about 90°. In embodiments where both shafts are formed with a curvature (of about 90°), the catheter assembly will be substantially straight when the shafts have been rotated through about 180° relative to one another. When the shafts have been rotated through about 90° relative to one another, the distal end of the catheter assembly will be disposed at an angle of approximately 45° to the proximal end thereof. However, in embodiments where slots are formed in one shaft, the catheter assembly will be substantially straight when the shafts have been rotated through about 90° relative to one another. When the shafts have been rotated through about 45° relative to one another the distal end of the catheter assembly will be disposed at an angle of approximately 45° to the proximal end thereof. Table A below sets out the angle of curvature of the catheter assembly for various embodiments of the present invention.

**Table A**

| **Inner Shaft** | **Outer Shaft** | **Relative rotation from aligned configuration** | |
|---|---|---|---|
| | | **90°** | **180°** |
| Formed with 90° curvature at distal end | Formed with 90° curvature at distal end | Catheter assembly distal end disposed at 45° to proximal end | Catheter assembly substantially straight |
| Formed with 90° curvature at distal end | Not formed with curvature, but formed with slots to allow bending in one plane | Catheter assembly substantially straight | Catheter assembly disposed at 90° in opposite direction to aligned configuration |
| Not formed with curvature, but formed with slots to allow bending in one plane | Formed with 90° curvature at distal end | Catheter assembly substantially straight | Catheter assembly disposed at 90° in opposite direction to aligned configuration |
| Not formed with curvature, but formed with slots to allow bending in one plane | Not formed with curvature, but formed with slots to allow bending in one plane | Catheter assembly substantially straight, flexibility minimised | Catheter assembly substantially straight, flexibility maximised |

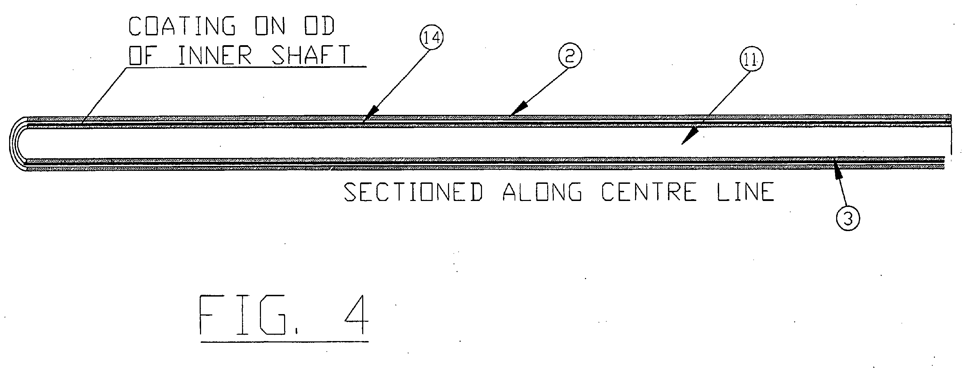

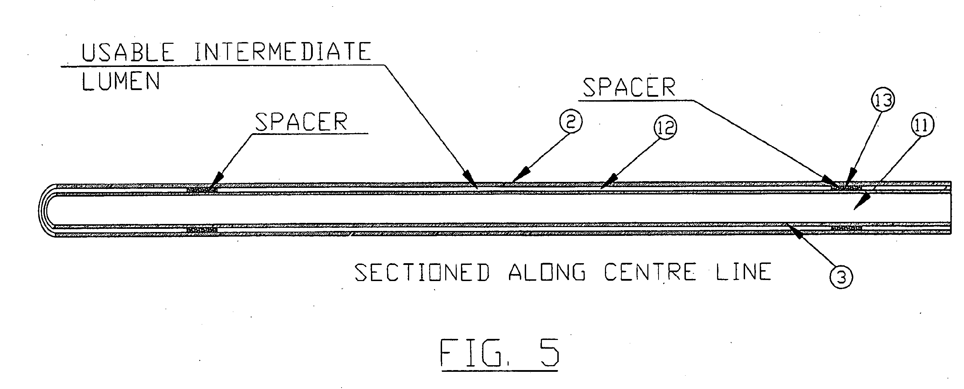

Referring now to Figures 4 and 5, it will be appreciated by those skilled in the art that an important advantage of the steerable catheter assembly of the present invention is that a working lumen 11 may be provided within the inner shaft of the catheter assembly. The inner working lumen 11 may be used, for example, to track over a guidewire, to inject contrast media, to deliver an endoscope to a site within the body or to supply inflation fluid to a catheter balloon. The working lumen may also be used for a variety of other applications.

In one embodiment of the present invention, as shown in Figure 5, an intermediate lumen 12 is provided between the inner and outer shafts 3,2. Spacers 13 are positioned between the inner and outer shafts to provide an intermediate working lumen 12. In the embodiment shown, the spacers 13 are substantially annular, but are not continuous around the entire circumference of the inner shaft. In an alternative embodiment, the spacers 13 are formed with through-holes (in a direction substantially parallel to the longitudinal axis of the catheter assembly). This ensures that fluid and/or devices may pass through the intermediate lumen. The wall thickness of the spacers 13 determines the size of the intermediate lumen 12. The spacers are pushed onto the outer wall of the inner shaft 2 before assembly of the steerable catheter. The spacer has the additional advantage of maintaining concentricity between the inner and outer shafts. The intermediate working lumen 12 may be used for any of the applications described above with reference to the inner working lumen 11. Additionally, in conjunction with the inner working lumen, the catheter assembly can be used, for example, to simultaneously track and deliver contrast media.

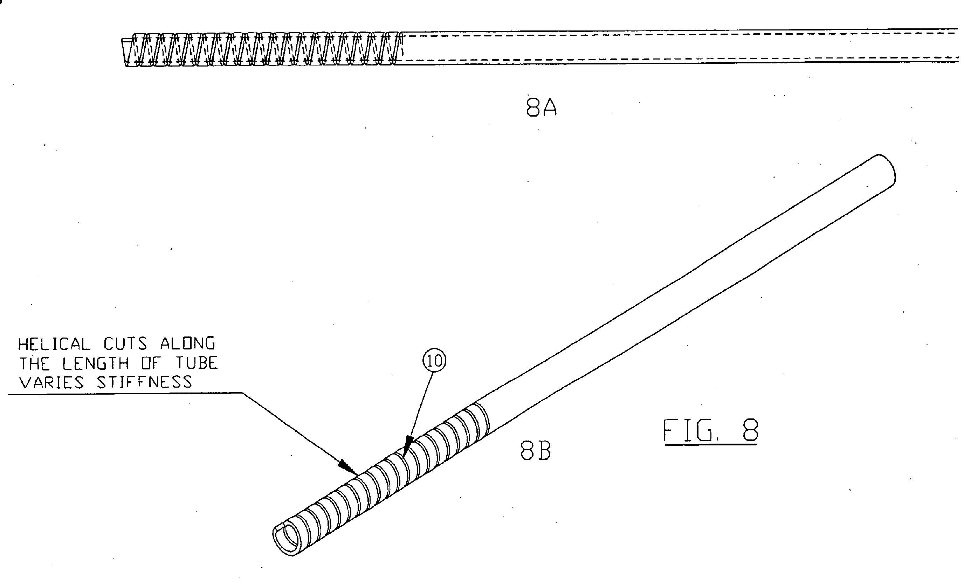

As shown in Figure 4 of the drawings, the inner shaft 3 may be provided with a coating 14. The coating serves to reduce frictional forces between the inner and outer shafts. In the embodiment shown in Figure 4, a coating 14 is applied to the outer wall of the inner shaft 2. However, in alternative embodiments, the coating 14 may be applied to the inner wall of the outer shaft 2 or to both shafts. The coating may comprise, for example, an extruded polymer overjacket, a heat shrink polymer overjacket, a dipped coating, a sprayed coating or any type of coating known in the art. The coating 14 may be hydrophobic or hydrophilic in nature, depending on the end application of the catheter assembly. In the embodiments shown in Figures 7 to 10, where slots are formed in one or both shafts, a polymer overjacket coating may be used to seal the lumen within the shaft, so that it becomes fluid tight.

In all of the embodiments described above, a handle is provided to control the steering mechanism of the catheter assembly. The handle is connected to the inner and outer shafts, such that relative rotation between the shafts may be effected. In certain embodiments, the handle is also arranged to allow relative longitudinal displacement between the inner and outer shafts.

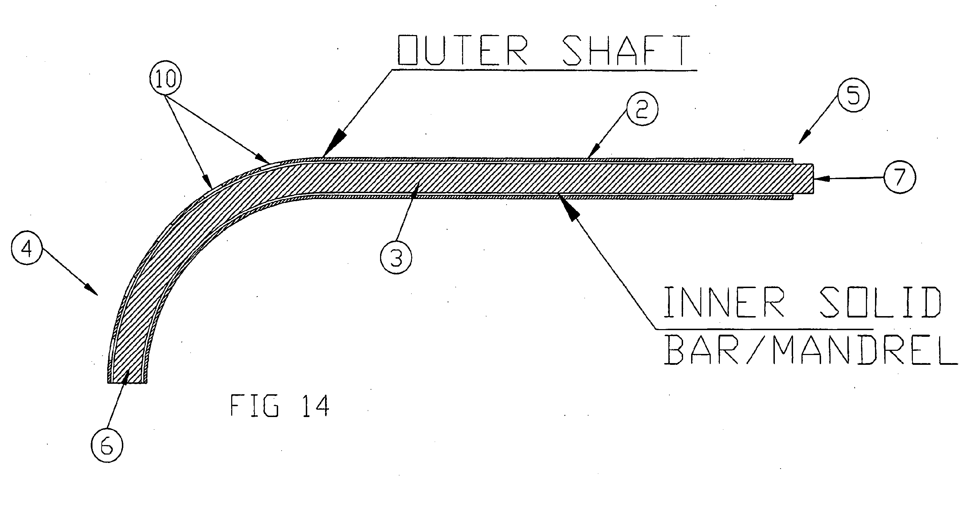

Referring now to Figure 14 of the drawings, there is provided a steerable catheter assembly 1, comprising an elongate outer shaft 2 having a lumen therethrough and having a proximal end 5 and a distal end 4; an elongate inner shaft 3 coaxially disposed within the lumen of the outer shaft and having a proximal end 7 and a distal end 6. The inner shaft 3 comprises a flexible solid bar or mandrel and is formed with a curvature at a distal portion 6 as described above. The outer shaft 2 is not pre-formed with a bend or curvature but has had material removed at a distal portion 4 to form slots 10 in the shaft 2, as described above with reference to Figure 9. The slots may be formed in one or alternatively both sides of the shaft. Suitably, the solid bar is made from a shaped memory metal, such as nitinol. Nickle-based, copper-based, iron-based, platinum-based or polymer shape memory materials may also be used.

In a first configuration shown in Figure 14, the distal portion 4 of the outer shaft 2 is substantially aligned with the distal portion 6 of the inner shaft 3, such that the catheter assembly 1 has a curvature of approximately 90 degrees at its distal end 8. In the present embodiment, the catheter assembly may be adjusted between angles of 0 degrees and 90 degrees. In alternative embodiments, other angles of curvature may be used.

As previously described, the steering mechanism works by effecting relative rotational displacement between the inner and outer shafts. In the 'at rest' position, the catheter assembly distal end 8 is therefore disposed at an angle of approximately 90 degrees to the catheter assembly proximal end 9. In this embodiment, the outer shaft 2 is clamped at its proximal end 5 so that it cannot rotate, whereas the inner shaft 3 is freely rotatable. When the inner shaft is rotated relative to the outer shaft, the outer shaft cannot deflect in any plane other than the single direction permitted by the slots, and the outer shaft thus begins to straighten. If the inner shaft is rotated through about 90° degrees, it exerts a deflection force on the outer shaft in the direction of curvature of the inner shaft. However, the rigidity of the outer shaft ensures that the outer shaft cannot be deflected (in any plane other than that allowed by the slots) beyond a straight position.

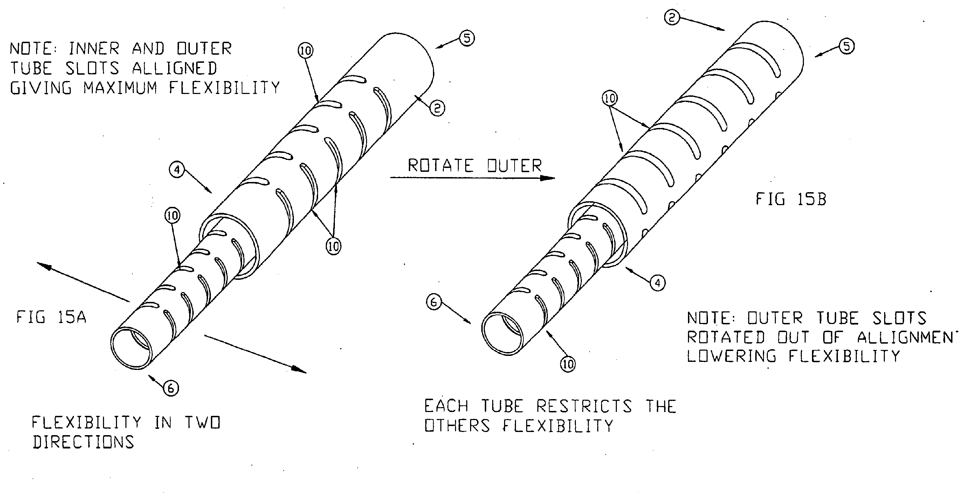

Referring now to Figure 15A and Figure 15B of the drawings, there is provided a steerable catheter assembly according to a second aspect of the invention, comprising an elongate outer shaft 2 having a lumen therethrough and having a proximal end 5 and a distal end 4 and an elongate inner shaft 3 coaxially disposed within the lumen of the outer shaft and having a proximal end 7 and a distal end 6. The distal portion 6 of the inner shaft 3 is capable of bending in a pre-determined bend plane only, and the distal portion 4 of the outer shaft 2 is capable of bending in a pre-determined bend plane only. The outer and inner shafts have had material removed at a distal portion to form slots 10, as described above with reference to Figures 9 and 10. The slots may be formed in one side of the shaft or more typically in two opposite side of the shafts. In alternative embodiments, other slot configurations may be used.

In an aligned configuration as shown in Figure 15A, the bend plane of the distal portion of the inner shaft is substantially aligned with the bend plane of the distal portion of the outer shaft, such that a distal end of the catheter assembly is capable of bending in the pre-determined bend plane with a maximum flexibility. As shown in Figure 15B, the inner shaft and the outer shaft are rotatable relative to one another out of the aligned configuration so that the bend plane of the distal portion of the inner shaft is moved out of alignment with the bend plane of the distal portion of the outer shaft to vary the flexibility of the distal end of the catheter assembly between the maximum flexibility and a minimum flexibility. Thus, rotating one shaft relative to the other results in a stiffening of the catheter assembly.

The words "comprises/comprising" and the words "having/including" when used herein with reference to the present invention are used to specify the presence of stated features, integers, steps or components but does not preclude the presence or addition of one or more other features, integers, steps, components or groups thereof.

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable sub-combination.

## Claims

1. A steerable catheter assembly (1), comprising:
an elongate outer shaft (2) having a lumen therethrough and having a proximal end (5) and a distal end (4);
an elongate inner shaft (3) coaxially disposed within the lumen of the outer shaft and having a proximal end (7) and a distal end (6); and
wherein at least one of the inner shaft and the outer shaft is formed with a curvature at a distal portion (4, 6), and **characterised in that** the distal portion (6, 4) of the other shaft is adapted to bend in a pre-determined bend plane only, and wherein the distal portion of the inner shaft is arranged within the distal portion of the outer shaft, such that in an aligned configuration, the curvature of the distal portion of the curved shaft is substantially aligned with the bend plane of the distal portion of the other shaft, such that a distal end (8) of the catheter assembly is disposed at a maximum deflection angle α to a longitudinal axis of the catheter assembly; and
the inner shaft (3) and the outer shaft (2) are rotatable relative to one another out of the aligned configuration so that the distal portion (4, 6) of the curved shaft is moved out of alignment with the bend plane of the distal portion (6, 4) of the other shaft such that each shaft exerts a deflection force on the other shaft to deflect the distal end (8) of the catheter assembly (1) between α and 0 degrees to the longitudinal axis of the catheter assembly.

2. A steerable catheter assembly as claimed in claim 1, **characterised in that** the inner shaft (3) includes an inner lumen (11) extending therethrough.

3. A steerable catheter assembly as claimed in any preceding claim, **characterised in that:**
the inner shaft (3) is formed with a curvature at a distal portion (6), and the outer shaft (2) is formed with a curvature at a distal portion (4), and in the aligned configuration, the distal portion (6) of the inner shaft is substantially aligned with the distal portion of the outer shall (4), such that the distal end (8) of the catheter assembly (1) is disposed at a maximum deflection angle α to a longitudinal axis of the catheter assembly; and
the inner shaft (3) and the outer shaft (2) are rotatable through an angle of rotation which may be in the range of 0° and about 180° relative to one another so that the distal portion (6) of the inner shaft (3) is moved out of alignment with the distal portion (4) of the outer shaft (2), such that each shaft exerts a deflection force on the other shaft to deflect the distal end (8) of the catheter assembly between α and 0 degrees to the longitudinal axis of the catheter assembly.

4. A steerable catheter assembly as claimed in claim 1, **characterised in that:**
the inner shaft (3) is formed with a curvature at a distal portion (6), and the distal portion (4) of the outer shaft (2) is capable of bending in a pre-determined bend plane only, and in the aligned configuration, the curvature of the distal portion (6) of the inner shaft (3) is substantially aligned with the bend plane of the distal portion (4) of the outer shaft (2), such that the distal end (8) of the catheter assembly is disposed at a maximum deflection angle α to a longitudinal axis of the catheter assembly; and
the inner shaft (3) and the outer shaft (2) are rotatable between 0 and 90 degrees relative to one another so that the distal portion (6) of the inner shaft (3) is moved out of alignment with the bend plane of the distal portion (4) of the outer shaft (2) to deflect the distal end of the catheter assembly (1) between α and 0 degrees to the longitudinal axis of the catheter assembly.

5. A steerable catheter assembly as claimed in claim 4, **characterised in that** a plurality of slots (10) are formed in a portion of the wall of the outer shaft (2) in a substantially spiral or circumferential path to allow a distal portion (4) of the outer shaft (2) to bend in a pre-determined bend plane only.

6. A steerable catheter assembly as claimed in claim 1, **characterised in that**:
the outer shaft (2) is formed with a curvature at a distal portion (4), and the distal portion (6) of the inner shaft (3) is capable of bending in a pre-determined bend plane only, and in the aligned configuration, the curvature of the distal portion (4) of the outer shaft (2) is substantially aligned with the bend plane of the distal portion (6) of the inner shaft (3), such that the distal end (8) of the catheter assembly (1) is disposed at a maximum deflection angle α to a longitudinal axis of the catheter assembly; and
the inner shaft (3) and the outer shaft (2) are rotatable between 0 and 90 degrees relative to one another so that the distal portion (4) of the outer shaft (2) is moved out of alignment with the bend plane of the distal portion (6) of the inner shaft (3) to deflect the distal end (8) of the catheter assembly (1) between α and 0 degrees to the longitudinal axis of the catheter assembly.

7. A steerable catheter assembly as claimed in claim 6, **characterised in that** a plurality of slots (10) are formed in a portion of the wall of the inner shaft (3) in a substantially spiral or circumferential path to allow a distal portion of the inner shaft to bend in a pre-determined bend plane only.

8. A steerable catheter assembly as claimed in any preceding claim, further **characterised in that** the outer shall (2) and the inner shaft (3) are displaceable longitudinally relative to one another out of the aligned configuration, such that each shaft exerts a deflection force on the other shaft to deflect the distal end (8) of the catheter assembly (1) between α and 0 degrees to the longitudinal axis of the catheter assembly.

9. A steerable catheter assembly as claimed in any preceding claim, wherein the outer shaft (2) is formed from stainless steel.

10. A steerable catheter assembly as claimed in any preceding claim, wherein the inner shaft (3) is formed from stainless steel.

11. A steerable catheter assembly as claimed in any preceding claim, **characterised in that** the distal portion (6) of the inner shaft (3), or the distal portion (4) of the outer shaft (2), or the distal portions of both shafts comprises a shape memory material.

12. A steerable catheter assembly as claimed in any preceding claim, **characterised in that** the distal portion (6) of the inner shaft (3), or the distal portion (4) of the outer shaft (2) or the distal portion of both shafts comprises a metal coil.

13. A steerable catheter assembly as claimed in any preceding claim, **characterised in that** at least one spacer (13) is disposed in the lumen of the outer shaft (2) between the outer shaft and the inner shaft such that an intermediate working lumen (12) is established therebetween.

14. A steerable catheter assembly, comprising:
an elongate outer shaft (2) having a lumen therethrough and having a proximal end (5) and a distal end (4);
an elongate inner shaft (3) coaxially disposed within the lumen of the outer shaft and having a proximal end (7) and a distal end (6); and
**characterised in that** the distal portion (6) of the inner shaft (3) is adapted to bend in one pre-determined bend plane only and the distal portion (4) of the outer shaft (2) is adapted to bend in one pre-determined bend plane only, the distal portion of the inner shaft is arranged within the distal portion of the outer shaft, and in an aligned configuration, the one bend plane of the distal portion (6) of the inner shaft (3) is substantially aligned with the one bend plane of the distal portion of the outer shaft (2), such that a distal end (8) of the catheter assembly (1) is capable of bending in the pre-determined bend plane with a maximum flexibility; and
the inner shaft (3) and the outer shaft (2) are rotatable relative to one another out of the aligned configuration so that the one bend plane of the distal portion of the inner shaft (3) is moved out of alignment with the one bend plane of the distal portion of the outer shaft (2) to vary the flexibility of the distal end (8) of the catheter assembly between the maximum flexibility and a minimum flexibility.

15. A steerable catheter assembly as claimed in claim 14, **characterised in that** a plurality of slots (10) are formed in a portion of the wall of the outer shaft (2) in a substantially spiral or circumferential path to allow a distal portion of the outer shaft to bend in a pre-determined bend plane only.

16. A steerable catheter assembly as claimed in claim 14 or 15, **characterised in that** a plurality of slots (10) are formed in a portion of the wall of the inner shaft in a substantially spiral or circumferential path to allow a distal portion of the inner shaft to bend in a pre-determined bend plane only.

## Patentansprüche

1. Lenkbare Katheteranordnung (1), umfassend:
- einen länglichen äußeren Schaft (2), welcher ein ihn durchsetzendes Lumen und ein nahes Ende (5) sowie ein fernes Ende (4) aufweist;
- einen länglichen inneren Schaft (3), welcher koaxial im Innern des Lumens des äußeren Schaftes angeordnet ist und ein nahes Ende (7) sowie ein fernes Ende (6) aufweist; und
- wobei wenigstens einer des inneren und des äußeren Schaftes an einem fernen Abschnitt (4, 6) mit einer Krümmung ausgebildet ist;
**dadurch gekennzeichnet, dass**
- der ferne Abschnitt (6, 4) des anderen Schaftes zur Biegung nur in einer vorherbestimmten Biegeebene ausgebildet ist, wobei der ferne Abschnitt des inneren Schaftes derart im Innern des fernen Abschnittes des äußeren Schaftes angeordnet ist, dass bei einer fluchtenden Anordnung die Krümmung des fernen Abschnittes des gekrümmten Schaftes im Wesentlichen mit der Biegeebene des fernen Abschnittes des anderen Schaftes fluchtet, so dass ein fernes Ende (8) der Katheteranordnung unter einem maximalen Ablenkwinkel α in Bezug auf eine Längsachse der Katheteranordnung angeordnet ist; und
- der innere Schaft (3) und der äußere Schaft (2) aus der fluchtenden Anordnung heraus relativ zueinander drehbar sind, so dass der ferne Abschnitt (4, 6) des gekrümmten Schaftes derart aus der fluchtenden Anordnung mit der Biegeebene des fernen Abschnittes (6, 4) des anderen Schaftes heraus bewegbar ist, dass jeder Schaft eine Ablenkkraft auf den anderen Schaft bewirkt, um das ferne Ende (8) der Katheteranordnung (1) zwischen einem Winkel α und 0° in Bezug auf die Längsachse der Katheteranordnung abzulenken.

2. Lenkbare Katheteranordnung nach Anspruch 1, **dadurch gekennzeichnet, dass** der innere Schaft (3) ein ihn durchsetzendes inneres Lumen (11) aufweist.

3. Lenkbare Katheteranordnung nach einem der vorangehenden Ansprüche, **dadurch gekennzeichnet, dass:**
- der innere Schaft (3) an einem fernen Abschnitt (6) mit einer Krümmung ausgebildet ist und der äußere Schaft (2) an einem fernen Abschnitt (4) mit einer Krümmung ausgebildet ist, wobei bei der fluchtenden Anordnung der ferne Abschnitt (6) des inneren Schaftes im Wesentlichen mit dem fernen Abschnitt (4) des äußeren Schaftes fluchtet, so dass das ferne Ende (8) der Katheteranordnung (1) unter einem maximalen Ablenkwinkel α in Bezug auf eine Längsachse der Katheteranordnung angeordnet ist; und
- der innere Schaft (3) und der äußere Schaft (2) um einen Drehwinkel, welcher im Bereich von 0° bis etwa 180° betragen kann, relativ zueinander drehbar sind, so dass der ferne Abschnitt (6) des inneren Schaftes (3) derart aus der fluchtenden Anordnung mit dem fernen Abschnitt (4) des äußeren Schaftes (2) heraus bewegbar ist, dass jeder Schaft eine Ablenkkraft auf den anderen Schaft bewirkt, um das ferne Ende (8) der Katheteranordnung zwischen einem Winkel α und 0° in Bezug auf die Längsachse der Katheteranordnung abzulenken.

4. Lenkbare Katheteranordnung nach Anspruch 1, **dadurch gekennzeichnet, dass:**
- der innere Schaft (3) an einem fernen Abschnitt (6) mit einer Krümmung ausgebildet ist und der ferne Abschnitt (4) des äußeren Schaftes (2) nur zur Biegung in einer vorherbestimmten Biegeebene ausgebildet ist, wobei bei der fluchtenden Anordnung die Krümmung des fernen Abschnittes (6) des inneren Schaftes (3) im Wesentlichen mit der Biegeebene des fernen Abschnittes (4) des äußeren Schaftes (2) fluchtet, so dass das ferne Ende (8) der Katheteranordnung unter einem maximalen Ablenkwinkel α in Bezug auf eine Längsachse der Katheteranordnung angeordnet ist; und
- der innere Schaft (3) und der äußere Schaft (2) zwischen 0° und 90° relativ zueinander drehbar sind, so dass der ferne Abschnitt (6) des inneren Schaftes (3) aus der fluchtenden Anordnung mit der Biegeebene des fernen Abschnittes (4) des äußeren Schaftes (2) heraus bewegbar ist, um das ferne Ende der Katheteranordnung (1) zwischen einem Winkel α und 0° in Bezug auf die Längsachse der Katheteranordnung abzulenken.

5. Lenkbare Katheteranordnung nach Anspruch 4, **dadurch gekennzeichnet, dass** an einem Abschnitt der Wandung des äußeren Schaftes (2) eine Mehrzahl an Schlitzen (10) gemäß einem im Wesentlichen spiralförmigen oder umfänglichen Erstreckungspfad gebildet sind, um dem fernen Abschnitt (4) des äußeren Schaftes (2) eine Biegung nur in einer vorherbestimmten Biegeebene zu ermöglichen.

6. Lenkbare Katheteranordnung nach Anspruch 1, **dadurch gekennzeichnet, dass:**
- der äußere Schaft (2) an einem fernen Abschnitt (4) mit einer Krümmung ausgebildet ist und der ferne Abschnitt (6) des inneren Schaftes (3) zur Biegung nur in einer vorherbestimmten Biegeebene ausgebildet ist, wobei bei der fluchtenden Anordnung die Krümmung des fernen Abschnittes (4) des äußeren Schaftes (2) im Wesentlichen mit der Biegeebene des fernen Abschnittes (6) des inneren Schaftes (3) fluchtet, so dass das ferne Ende (8) der Katheteranordnung (1) unter einem maximalen Ablenkwinkel α in Bezug auf eine Längsachse der Katheteranordnung angeordnet ist; und
- der innere Schaft (3) und der äußere Schaft (2) zwischen 0° und 90° relativ zueinander drehbar sind, so dass der ferne Abschnitt (4) des äußeren Schaftes (2) aus der fluchtenden Anordnung mit der Biegeebene des fernen Abschnittes (6) des inneren Schaftes (3) heraus bewegbar ist, um das ferne Ende (8) der Katheteranordnung (1) zwischen einem Winkel α und 0° in Bezug auf die Längsachse der Katheteranordnung abzulenken.

7. Lenkbare Katheteranordnung nach Anspruch 6, **dadurch gekennzeichnet, dass** an einem Abschnitt der Wandung des inneren Schaftes (3) eine Mehrzahl an Schlitzen (10) gemäß einem im Wesentlichen spiralförmigen oder umfänglichen Erstreckungspfad gebildet sind, um dem fernen Abschnitt des inneren Schaftes eine Biegung nur in einer vorherbestimmten Biegeebene zu ermöglichen.

8. Lenkbare Katheteranordnung nach einem der vorangehenden Ansprüche, ferner **dadurch gekennzeichnet, dass** der äußere Schaft (2) und der innere Schaft (3) aus der fluchtenden Anordnung heraus in Längsrichtung relativ zueinander verschiebbar sind, so dass jeder Schaft eine Ablenkkraft auf den anderen Schaft bewirkt, um das ferne Ende (8) der Katheteranordnung (1) zwischen einem Winkel α und 0° in Bezug auf die Längsachse der Katheteranordnung abzulenken.

9. Lenkbare Katheteranordnung nach einem der vorangehenden Ansprüche, wobei der äußere Schaft (2) aus rostfreiem Stahl gefertigt ist.

10. Lenkbare Katheteranordnung nach einem der vorangehenden Ansprüche, wobei der innere Schaft (3) aus rostfreiem Stahl gefertigt ist.

11. Lenkbare Katheteranordnung nach einem der vorangehenden Ansprüche, **dadurch gekennzeichnet, dass** das ferne Ende (6) des inneren Schaftes (3) oder das ferne Ende (4) des äußeren Schaftes (2) oder die fernen Enden beider Schäfte ein Formgedächtnismaterial enthalten.

12. Lenkbare Katheteranordnung nach einem der vorangehenden Ansprüche, **dadurch gekennzeichnet, dass** das ferne Ende (6) des inneren Schaftes (3) oder das ferne Ende (4) des äußeren Schaftes (2) oder die fernen Enden beider Schäfte eine Metallfeder enthalten.

13. Lenkbare Katheteranordnung nach einem der vorangehenden Ansprüche, **dadurch gekennzeichnet, dass** in dem Lumen des äußeren Schaftes (2) zwischen dem äußeren Schaft und dem inneren Schaft wenigstens ein Abstandhalter (13) angeordnet ist, so dass zwischen denselben ein dazwischen liegendes Arbeitslumen (12) gebildet ist.

14. Lenkbare Katheteranordnung, umfassend:
- einen länglichen äußeren Schaft (2), welcher ein ihn durchsetzendes Lumen und ein nahes Ende (5) sowie ein fernes Ende (4) aufweist;
- einen länglichen inneren Schaft (3), welcher koaxial im Innern des Lumens des äußeren Schaftes angeordnet ist und ein nahes Ende (7) sowie ein fernes Ende (6) aufweist;
**dadurch gekennzeichnet, dass**
- der ferne Abschnitt (6) des inneren Schaftes (3) zur Biegung nur in einer vorherbestimmten Biegeebene ausgebildet ist und der ferne Abschnitt (4) des äußeren Schaftes (2) zur Biegung nur in einer vorherbestimmten Biegeebene ausgebildet ist, wobei der ferne Abschnitt des inneren Schaftes im Innern des fernen Abschnittes des äußeren Schaftes angeordnet ist, wobei bei einer fluchtenden Anordnung die eine Biegeebene des fernen Abschnittes (6) des inneren Schaftes (3) im Wesentlichen mit der einen Biegeebene des fernen Abschnittes des äußeren Schaftes (2) fluchtet, so dass ein fernes Ende (8) der Katheteranordnung (1) zur Biegung in einer vorherbestimmten Biegeebene mit einer maximalen Nachgiebigkeit in der Lage ist; und
- der innere Schaft (3) und der äußere Schaft (2) aus der fluchtenden Anordnung heraus relativ zueinander drehbar sind, so dass die eine Biegeebene des fernen Abschnittes des inneren Schaftes (3) aus der fluchtenden Anordnung mit der einen Biegeebene des fernen Abschnittes des äußeren Schaftes (2) heraus bewegbar ist, um die Nachgiebigkeit des fernen Endes (8) der Katheteranordnung zwischen der maximalen Nachgiebigkeit und einer minimalen Nachgiebigkeit zu verändern.

15. Lenkbare Katheteranordnung nach Anspruch 14, **dadurch gekennzeichnet, dass** an einem Abschnitt der Wandung des äußeren Schaftes (2) eine Mehrzahl an Schlitzen (10) gemäß einem im Wesentlichen spiralförmigen oder umfänglichen Erstreckungspfad gebildet sind, um dem fernen Abschnitt des äußeren Schaftes eine Biegung nur in einer vorherbestimmten Biegeebene zu ermöglichen.

16. Lenkbare Katheteranordnung nach Anspruch 14 oder 15, **dadurch gekennzeichnet, dass** an einem Abschnitt der Wandung des inneren Schaftes eine Mehrzahl an Schlitzen gemäß einem im Wesentlichen spiralförmigen oder umfänglichen Erstreckungspfad gebildet sind, um dem fernen Abschnitt des inneren Schaftes eine Biegung nur in einer vorherbestimmten Biegeebene zu ermöglichen.

## Revendications

1. Ensemble de cathéter dirigeable (1), comprenant :
une tige externe allongée (2) ayant une lumière à travers celle-ci et ayant une extrémité proximale (5) et une extrémité distale (4) ;
une tige interne allongée (3) disposée de manière coaxiale à l'intérieur de la lumière de la tige externe et ayant une extrémité proximale (7) et une extrémité distale (6) ; et
dans lequel au moins l'une parmi la tige interne et la tige externe est formée avec une courbure au niveau de la partie distale (4, 6) et **caractérisé en ce que** la partie distale (6, 4) de l'autre tige est adaptée pour se fléchir uniquement dans un plan de flexion prédéterminé, et dans lequel la partie distale de la tige interne est agencée à l'intérieur de la partie distale de la tige externe, de sorte que dans une configuration alignée, la courbure de la partie distale de la tige courbée est sensiblement alignée avec le plan de flexion de la partie distale de l'autre tige, de sorte qu'une extrémité distale (8) de l'ensemble de cathéter est disposée selon un angle de déflexion maximum α par rapport à un axe longitudinal de l'ensemble de cathéter ; et
la tige interne (3) et la tige externe (2) peuvent tourner l'une par rapport à l'autre hors de la configuration alignée de sorte que la partie distale (4, 6) de la tige incurvée est retirée de l'alignement avec le plan de flexion de la partie distale (6, 4) de l'autre tige de sorte que chaque tige exerce une force de déflexion sur l'autre tige afin de dévier l'extrémité distale (8) de l'ensemble de cathéter (1) entre α et 0 degrés par rapport à l'axe longitudinal de l'ensemble de cathéter.

2. Ensemble de cathéter dirigeable selon la revendication 1, **caractérisé en ce que** la tige interne (3) comprend une lumière interne (11) s'étendant à travers celle-ci.

3. Ensemble de cathéter dirigeable selon l'une quelconque des revendications précédentes, **caractérisé en ce que** :
la tige interne (3) est formée avec une courbure au niveau d'une partie distale (6), et la tige externe (2) est formée avec une courbure au niveau d'une partie distale (4) et dans la configuration alignée, la partie distale (6) de la tige interne est sensiblement alignée avec la partie distale de la tige externe (4), de sorte que l'extrémité distale (8) de l'ensemble de cathéter (1) est disposée selon un angle de déflexion maximum α par rapport à un axe longitudinal de l'ensemble de cathéter ; et
la tige interne (3) et la tige externe (2) peuvent tourner sur un angle de rotation qui peut être de l'ordre de 0° et d'environ 180° l'une par rapport à l'autre, de sorte que la partie distale (6) de la tige interne (3) est retirée de l'alignement avec la partie distale (4) de la tige externe (2), de sorte que chaque tige exerce une force de déflexion sur l'autre tige afin de dévier l'extrémité distale (8) de l'ensemble de cathéter entre α et 0 degrés par rapport à l'axe longitudinal de l'ensemble de cathéter.

4. Ensemble de cathéter dirigeable selon la revendication 1, **caractérisé en ce que :**
la tige interne (3) est formée avec une courbure au niveau d'une partie distale (6) et la partie distale (4) de la tige externe (2) peut se fléchir uniquement dans un plan de flexion prédéterminé, et dans la configuration alignée, la courbure de la partie distale (6) de la tige interne (3) est sensiblement alignée avec le plan de flexion de la partie distale (4) de la tige externe (2), de sorte que l'extrémité distale (8) de l'ensemble de cathéter est disposée selon un angle de déflexion maximum α par rapport à un axe longitudinal de l'ensemble de cathéter ; et
la tige interne (3) et la tige externe (2) peuvent tourner entre 0 et 90 degrés l'une par rapport à l'autre de sorte que la partie distale (6) de la tige interne (3) est retirée de l'alignement avec le plan de flexion de la partie distale (4) de la tige externe (2) afin de dévier l'extrémité distale de l'ensemble de cathéter (1) entre α et 0 degré par rapport à l'axe longitudinal de l'ensemble de cathéter.

5. Ensemble de cathéter dirigeable selon la revendication 4, **caractérisé en ce qu'**une pluralité de fentes (10) est formée sur une partie de la paroi de la tige externe (2) dans une trajectoire sensiblement en spirale ou circonférentielle afin de permettre la flexion d'une partie distale (4) de la tige externe (2) uniquement dans un plan de flexion prédéterminé.

6. Ensemble de cathéter dirigeable selon la revendication 1, **caractérisé en ce que :**
la tige externe (2) est formée avec une courbure au niveau d'une partie distale (4) et la partie distale (6) de la tige interne (3) peut se fléchir uniquement dans un plan de flexion prédéterminé, et dans la configuration alignée, la courbure de la partie distale (4) de la tige externe (2) est sensiblement alignée avec le plan de flexion de la partie distale (6) de la tige interne (3), de sorte que l'extrémité distale (8) de l'ensemble de cathéter (1) est disposée selon un angle de déflexion maximum α par rapport à un axe longitudinal de l'ensemble de cathéter ; et
la tige interne (3) et la tige externe (2) peuvent tourner entre 0 et 90 degrés l'une par rapport à l'autre de sorte que la partie distale (4) de la tige externe (2) est retirée de l'alignement avec le plan de flexion de la partie distale (6) de la tige interne (3) pour dévier l'extrémité distale (8) de l'ensemble de cathéter (1) entre α et 0 degré par rapport à l'axe longitudinal de l'ensemble de cathéter.

7. Ensemble de cathéter dirigeable selon la revendication 6, **caractérisé en ce qu'**une pluralité de fentes (10) est formée dans une partie de la paroi de la tige interne (3) dans une trajectoire sensiblement en spirale ou circonférentielle afin de permettre la flexion d'une partie distale de la tige interne uniquement dans un plan de flexion prédéterminé.

8. Ensemble de cathéter dirigeable selon l'une quelconque des revendications précédentes, **caractérisé en outre en ce que** la tige externe (2) et la tige interne (3) peuvent être déplacées longitudinalement l'une par rapport à l'autre hors de la configuration alignée, de sorte que chaque tige exerce une force de déflexion sur l'autre tige afin de dévier l'extrémité distale (8) de l'ensemble de cathéter (1) entre α et 0 degré par rapport à l'axe longitudinal de l'ensemble de cathéter.

9. Ensemble de cathéter dirigeable selon l'une quelconque de revendications précédentes, dans lequel la tige externe (2) est formée à partir d'acier inoxydable.

10. Ensemble de cathéter dirigeable selon l'une quelconque des revendications précédentes, dans lequel la tige interne (3) est formée à partir d'acier inoxydable.

11. Ensemble de cathéter dirigeable selon l'une quelconque des revendications précédentes, **caractérisé en ce que** la partie distale (6) de la tige interne (3) ou bien la partie distale (4) de la tige externe (2) ou bien les parties distales de deux tiges comprennent un matériau à mémoire de forme.

12. Ensemble de cathéter dirigeable selon l'une quelconque des revendications précédentes, **caractérisé en ce que** la partie distale (6) de la tige interne (3) ou bien la partie distale (4) de la tige externe (2) ou bien la partie distale de deux tiges comprennent une bobine métallique.

13. Ensemble de cathéter dirigeable selon l'une quelconque des revendications précédentes, **caractérisé en ce qu'**au moins un dispositif d'espacement (13) est disposé dans la lumière de la tige externe (2) entre la tige externe et la tige interne de sorte qu'une lumière de travail intermédiaire (12) est établie entre elles.

14. Ensemble de cathéter dirigeable, comprenant :
une tige externe allongée (2) ayant une lumière à travers celle-ci et ayant une extrémité proximale (5) et une extrémité distale (4) ;
une tige interne allongée (3) disposée de manière coaxiale à l'intérieur de la lumière de la tige externe et ayant une extrémité proximale (7) et une extrémité distale (6) ; et
**caractérisé en ce que** la partie distale (6) de la tige interne (3) est adaptée pour fléchir uniquement dans un plan de flexion prédéterminé et la partie distale (4) de la tige externe (2) est adaptée pour fléchir uniquement dans un plan de flexion prédéterminé, la partie distale de la tige interne est agencée à l'intérieur de la partie distale de la tige externe, et dans une configuration alignée, le plan de flexion de la partie distale (6) de la tige interne (3) est sensiblement aligné avec le plan de flexion de la partie distale de la tige externe (2), de sorte qu'une extrémité distale (8) de l'ensemble de cathéter (1) peut se fléchir dans le plan de flexion prédéterminé avec une flexibilité maximale ; et
la tige interne (3) et la tige externe (2) peuvent tourner l'une par rapport à l'autre hors de la configuration alignée de sorte que le plan de flexion de la partie distale de la tige interne (3) est retiré de l'alignement avec le plan de flexion de la partie distale de la tige externe (2) pour modifier la flexibilité de l'extrémité distale (8) de l'ensemble de cathéter entre la flexibilité maximum et une flexibilité minimum.

15. Ensemble de cathéter dirigeable selon la revendication 14, **caractérisé en ce qu'**une pluralité de fentes (10) est formée dans une partie de la paroi de la tige externe (2) dans une trajectoire sensiblement en spirale ou circonférentielle afin de permettre la flexion d'une partie distale de la tige externe uniquement dans un plan de flexion prédéterminé.

16. Ensemble de cathéter dirigeable selon la revendication 14 ou 15, **caractérisé en ce qu'**une pluralité de fentes (10) est formée dans une partie de la paroi de la tige interne dans une trajectoire sensiblement en spirale ou circonférentielle pour permettre de fléchir une partie distale de la tige externe uniquement dans un plan de flexion prédéterminé.
